# EUROPEAN PATENT APPLICATION

(11) **EP 4 755 903 A1**
(43) Date of publication of application: **10.06.2026**
(21) Application number: 24218142.8
(22) Date of filing: 06.12.2024
(51) Int. Cl.: C07K 14/36, C07K 5/00, C12N 15/52, C12P 19/38

(54) **GENETIC CONSTRUCTS FOR THE IMPROVED PRODUCTION OF MUREIDOMYCIN A**

(71) Applicant: Eberhard-Karls-Universität Tübingen, 72074 Tübingen (DE)
(72) Inventor: GUST, Bertolt, 72119 Ammerbuch (DE); HAUCK, Nils, 72076 Tübingen (DE); KOUTSANDREA, Panagiota-Hanna, 72074 Tübingen (DE); SONNENGRÜN, Richard, 72076 Tübingen (DE); GROSS, Harald, 72108 Rottenburg-Wurmlingen (DE)
(74) Representative: Kraus & Lederer PartGmbB

(57) **Abstract**

The present invention relates to a genetic construct comprising a genetically modified pacidamycin gene cluster pacNH01 and/or a genetically modified napsamycin npsXT01 gene cluster.

## Description

Mureidomycins are a subclass of uridyl peptide antibiotics (UPAs) which were originally isolated from *Streptomyces flavidovirens* and display potent and selective activity against the pathogen *Pseudomonas aeruginosa* which is a Gram-negative bacterium. Mureidomycin A (Fig. 1) has been described and claimed in US patent no. 5,213,974. The scientific article "MUREIDOMYCIN A~D, NOVEL PEPTIDYLNUCLEOSIDE ANTIBIOTICS" published by Isono et al., Journal of Antibiotics, May 1989, vol. XLII, No. 5, pages 674-679 describes mureidomycins which target bacterial cell wall biosynthesis by inhibiting phospho-MurNAc-pentapeptide translocase (MraY).

The biological data of mureidomycins, and mureidomycin A in particular, are highly promising since resistance of bacteria against more and more known antibiotic agents is an increasing problem. In the case of nosocomial infections like pneumonia or bacteremia, which occur especially in vulnerable patients, effective antibiotic agents are urgently needed. Moreover, ventilator-associated pneumonia in mechanically ventilated patients demands frequent antibiotic treatment to which nosocomial pathogens ought to be sensitive.

The herein discussed class of uridine-derived nucleosides consists of uridyl peptide antibiotics (UPAs). Four subclasses have been described encompassing the mureidomycins, napsamycins, pacidamycins and sansanmycins. Their target consists of phospho-N-acetylmuramyl-pentapeptide translocase (MraY) which constitutes a promising, underexplored antibiotic target, hitherto not addressed by antibiotics on the market. Mureidomycins were firstly isolated from *Streptomyces flavidovirens* SANK60486. The commercial use of mureidomycins has been hindered due to structural complexity and the resulting difficulties and expenses associated with chemical synthesis.

The biological synthesis via fermentation of suitable culture medium with a suitable *Streptomyces flavidovirens* strain bears the disadvantage that on the one hand mureidomycin A yield is very low and on the other hand several other uridine-derived nucleosides are produced in the fermentation broth. Isolation and purification of the individual compounds with high purity or homogeneity proves difficult in view of the structural similarity of the compounds.

It is therefore the objective of the present invention to provide means for an improved production process by genetic techniques and by selecting appropriate modifications which improve the yield of the desired compounds.

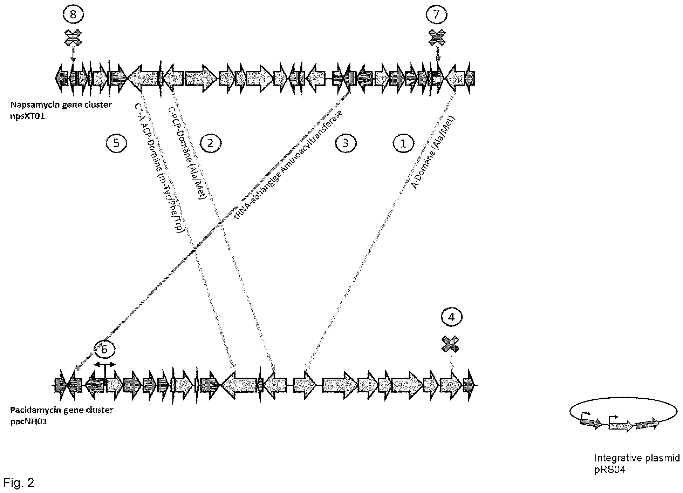

The biosynthetic gene clusters (BGC) of pacidamycins, napsamycins, sansanmycins and a cryptic mureidomycin gene cluster have been identified. The pacidamycin BGC from *S. coeruleorubidus* NRRL 18370 and AB1183F-64 was reported from two independent working groups in 2010 (Rackham, et al., Revealing the first uridyl peptide antibiotic biosynthetic gene cluster and probing pacidamycin biosynthesis, Bioeng. Bugs 2, 218-221 (2011) and Zhang, et al., Identification of the biosynthetic gene cluster for the pacidamycin group of peptidyl nucleoside antibiotics. Proc. Natl. Acad. Sci. 107, 16828-16833 (2010)). It consists of 22 open reading frames (ORFs), spanning over approximately 30.3 kb (Fig. 2). The napsamycin BGC was identified in *S*. sp. DSM5940 and described to span approximately 34.5 kb, distributed over 29 ORFS, and was reported to produce napsamycins and mureidomycins (Fig. 2) (Kaysser, et al., Identification of a napsamycin biosynthesis gene cluster by genome mining, Chembiochem Eur. J. Chem. Biol. 12, 477-487 (2011).

The pacidamycin (pacNH01) and napsamycin (npsXT01) biosynthetic gene clusters are shown in Figure 2 wherein the orthologous genes have been connected. In the following Table 1, the orthologous genes of the pacidamycin and napsamycin gene cluster and the corresponding postulated function are shown alongside the degree of identity and similarity.

**Table 1: Orthologous genes of the pacidamycin and napsamycin gene clusters and deduced roles, generated by BLAST analysis**

| **Gene** | **Postulated function** | **Napsamycin ortholog** | **Identity (%)** | **Similarity (%)** |
|---|---|---|---|---|
| *pac1 (A)* | Transcriptional regulator | *npsM* | 81 | 87 |
| *pac2 (B)* | tRNA-dependent aminoacyl transferase (R1: N-terminal _{L}-Ala) | *npsN* | 67 | 82 |
| *pac3 (C)* | Putative major facilitator transporter (UPA export) | *npsO* | 84 | 93 |
| *pac4 (D)* | NRPS condensation (C) domain (condensation of DABA with Pac14-tethered thioester of C-terminal ureido peptide) | *npsP7* | 68 | 77 |
| *pac5 (E)* | PLP-dependent aminotransferase (uridine modification: 5'-transamination) | *npsQ* | 87 | 93 |
| *pac6 (F)* | Fe(II)/α-oxoglutarate oxygenase (postulated uridine modification) | *npsR* | 72 | 82 |
| *pac7 (G)* | Oxidoreductase | *npsS* | 85 | 94 |
| *pac8 (H)* | NRPS thiolation (T) domain (key carrier domain of biosynthesis) | *npsP1* | 77 | 87 |
| *pac9 (I)* | NRPS C-domain (condensation of peptide chain with nucleoside substrate) | *npsP2* | 78 | 86 |
| *pac10 (J)* | MbtH domain (interaction with adenylation domains, specifically A-domain Pac12) | *npsO* | 66 | 80 |
| *pac11 (K)* | FAD-dependent oxidoreductase (glycolate oxidase, uridine modification: 5'-OH → 5'-aldehyde) | *npsE* | 81 | 88 |
| *pac12 (L)* | NRPS (C_{truncated}-A-T, activation of aromatic amino acids for position five of the UPA scaffold) | *npsP3* | 67 | 77 |
| *pac13 (M)* | Monomeric, metal-free cupin dehydratase (uridine modification: 3',4'-dehydration) | *npsF* | 77 | 90 |
| *pac14 (N)* | NRPS C-T didomain (Cognate loading partner of Pac15, catalyzation of ureido bond formation) | *npsP4* | 68 | 79 |
| *pac15 (O)* | NRPS A-domain (preferred activation of _{L}-Ala for position four of the UPA scaffold) | *npsP8* | 73 | 84 |
| *pac16 (P)* | NRPS A-T-TE tridomain (DABA activation) | *npsP5* | 84 | 90 |
| *pac17 (Q)* | Argininosuccinate lyase (DABA biosynthesis) | *npsG* | 87 | 91 |
| *pac18 (R)* | Homoserine kinase (DABA biosynthesis, putative threonine kinase might provide improved leaving group for β-elimination) | *npsH* | 86 | 92 |
| *pac19 (S)* | DABA synthase (Didomain protein containing a PLP-dependent cysteine synthase and an argininosuccinate lyase, catalyzes the β-replacement of the threonine hydroxyl by the α-amino of aspartate) | *npsI* | 85 | 91 |
| *pac20 (T)* | PLP-dependent threonine aldolase (responsible for 3S configuration of DABA) | *npsJ* | 87 | 92 |
| *pac21 (U)* | NRPS A-domain (dual function: specific activation of N-terminal alanine and condensation with DABA) | *npsP6* | 74 | 83 |
| *pac22 (V)* | SAM-dependent *N-*methyltransferase (*N-*methylation of DABA) | *npsV* | 74 | 84 |
| *pac21h (W)* | NRPS A-domain (specific activation of N-terminal *meta*-tyrosine) | *npsP6* | 77 | 86 |
| *phhA (X)* | Phenylalanine-3-hydroxylase (formation of *meta*-tyrosine from phenylalanine) | *npsK* | 78 | 85 |
| Not present in pac | Transcriptional regulator | *npsA* | - | - |
| Not present in pac | *N-acetyltransferase* | *npsB* | - | - |
| Not present in pac | Phosphatase | *npsC* | - | - |
| Not present in pac | Hypothetical protein | *npsL* | - | - |
| Not present in pac | Hypothetical protein | *npsT* | - | - |
| Not present in pac | FMN-dependent oxidoreductase (formation of dihydrouracil) | *npsU* | - | - |

The original sequences of the pacidamycin and napsamycin gene clusters were deposited with the NCBI accession numbers GU938463.1 and HQ287563.1, respectively.

A summary of the identification and characterization of enzymes involved in the biosynthesis of pyrimidine nucleoside antibiotics is disclosed in McErlean et al., Natural Product Reports, Vol. 38, issue 7, July 2021, pages 1362-1407. For the description and proper comprehension of the gene clusters we explicitly refer thereto.

For the expression of mureidomycin A, several *Streptomyces* strains were tested. These strains are summarized in Table 2.

**Table 2 Streptomyces strains**

| **Organism** | **Strain** | **Description** | **Reference** |
|---|---|---|---|
| *Streptomyces albus* | J1074 | WT, heterologous host (HH) | Chafer and Wilde (1976), J.Bacteriol. |
| *Streptomyces* sp. | DSM5940 | WT, napsamycin producer strain | Kaysser et al. (2011), Chem biochem. Eur.J.Chem., Biol, 12, pp477-487 |
| *Streptomyces albus* | Del14 | Derivative of *S*. *albus* J1074 with deletion of 15 biosynthetic gene clusters, heterologous host (HH) | Myronovskyi et al. (2018), Metab.Eng., 49, pp 316-324 |
| *Streptomyces albus* | B2P1 | Derivative of Dell4 with one additional *attB* site, heterologous host (HH) | Myronovskyi *et al*. (2018) |
| *Streptomyces griseus* | CS065 | WT, heterologous host (HH) | Malmierca et al. (2018), Front. Microbiol. 9, p 39 |
| *Streptomyces albus* | Del14/ pacHK11/pRS04 | HH strain with modified pacidamycin gene cluster pacHK11 and integrative plasmid pRS04 | Present work |
| *Streptomyces albus* | Del14/npsHK06 | HH strain with modified napsamycin gene cluster npsHK06 | Present work |

In view of the low production of mureidomycin A, it is an objective of the present invention to provide means and methods to address this issue which impedes further development and the subsequent commercial use of this compound as a novel drug candidate. Although the yield of antibiotics produced by *Streptomyces* strains is frequently improved by heterologous expression of entire biosynthetic gene clusters in non-modified heterologous hosts, such a method was not successful regarding mureidomycin A formation of the napsamycin gene cluster (npsXT01). More specifically, the production rate of mureidomycin A could not be increased significantly, while closely-related, but less active compounds were produced by the heterologous host in substantial amounts. This resulted in considerable difficulties concerning the production and isolation of homogenous mureidomycin A whereby the output should be a pure product.

The present invention relates therefore to the following preferred embodiments:
1. Genetic construct comprising the genetically modified pacidamycin gene cluster pacNH01 and/or a genetically modified napsamycin gene cluster npsXT01.
2. The genetic construct according to embodiment 1 which comprises further the modified pacidamycin gene cluster with an integrative plasmid pRS04 and the modified napsamycin gene cluster for improved mureidomycin A production.
3. The genetic construct of the present invention is preferably further characterized in that in the pacidamycin gene cluster *pac21* is knocked out, *pac15, pac14, pac2* and *pac12* were replaced with *npsP8, npsP4, npsN* and *npsP3,* respectively, and heterologous promoters P15 and P16 are inserted upstream of *pac3* and *pac4.* For the napsamycin biosynthetic gene cluster, *npsU* and *npsB* are knocked out.
4. The genetic construct according to the present invention is preferably part of a genetic vector, whereby the vector is preferably a plasmid, a cosmid or a fosmid. Such vector allows the transfer of the genetic construct in a suitable bacterial strain which is preferably a *Streptomyces* strain.
5. The genetic construct according to the invention is preferably expressed in a *Streptomyces* strain which contains a genetic construct according to the invention resulting in an improved production of mureidomycin A.
6. Among the useable *Streptomyces* strains a *Streptomyces albus* strain is particularly preferred.
7. Also particularly preferred is an Actinomycetes strain which contains a genetic construct according to embodiment 1-3.

Preferred embodiments of the present invention are described in more detail in the claims and the Figures of the present application.

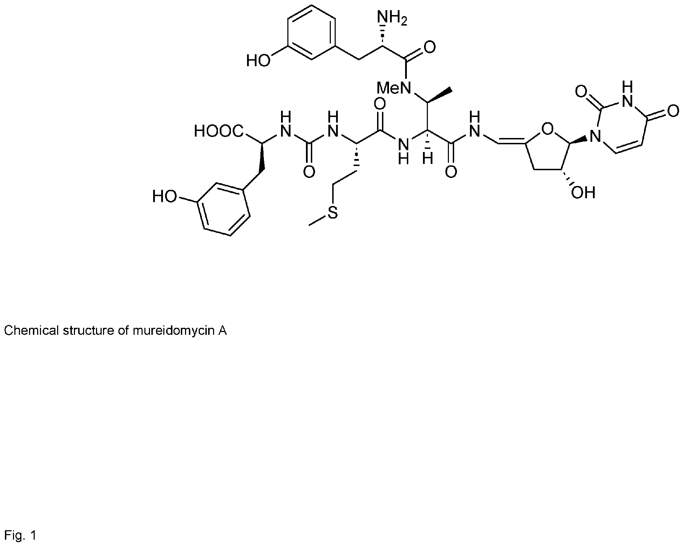

Figure 1 shows the chemical structure of mureidomycin A.
Figure 2 shows the napsamycin gene cluster npsXT01 and the pacidamycin gene cluster pacNH01 whereby the function of the individual genes is coded as follows:
   Function of the individual genes color-coded.
   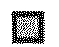 Genes for regulation and resistance - responsible for transcription strength and protection of the host cell
   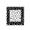 Nonribosomal peptide synthetase (NRPS) - biosynthetic genes of the peptide chain
   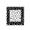 DABA biosynthesis - biosynthetic genes for the central DABA moiety starting from L-threonine
   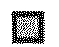 Synthesis of building blocks and tailoring reactions - e.g.: methylation, glycination, oxidation, reduction
   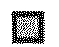 Unknown function
   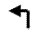 Synthetic promoters - inserted synthetic promoters with influence on transcription
   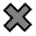 Deleted genes - genes that have been removed to abolish their function

Moreover, Figure 2 shows a detailed description of the modifications regarding the UPA gene clusters related to the pacidamycin gene cluster. Numbers 1-6 relate to the pacidamycin gene cluster and have the following meaning:

### Detailed description of modifications regarding the UPA gene clusters

Related to the pacidamycin gene cluster: (Figure 2)
1. Replacement of a gene in order to produce derivatives with methionine at amino acid position 4 (AA4).
2. Replacement of a gene in order to produce derivatives with methionine at amino acid position 4 (AA4) more specifically.
3. Replacement of a gene in order to produce derivatives with glycine at amino acid position 1 (AA1).
4. Deletion of a gene in order to eliminate production of derivatives carrying alanine at position 2 (AA2). Introduction of an integrative plasmid to increase transcription, the pool of the biosynthetic precursor m-tyrosine and to complement the abovementioned gene deletion with a homologue - Leads to the production of derivatives with m-tyrosine at position 2 (AA2).
5. Replacement of a gene in order to produce derivatives with m-tyrosine at position 5 (AA5) in a more targeted manner.
6. Introduction of an artificial bidirectional promoter to increase the production of all derivatives.

Concerning the napsamycin gene cluster numbers 7 and 8 have the following meaning:
7. Deletion of a gene in order to eliminate production of mureidomycin derivatives harboring a double bond at the uridine residue.
8. Deletion of a gene in order to eliminate production of mureidomycin derivatives harboring an acetyl group at the N-terminus.

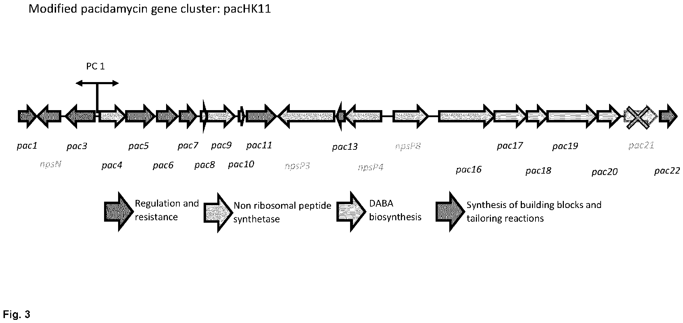

Figure 3 shows the modified pacidamycin cluster having the designation pacHK11.

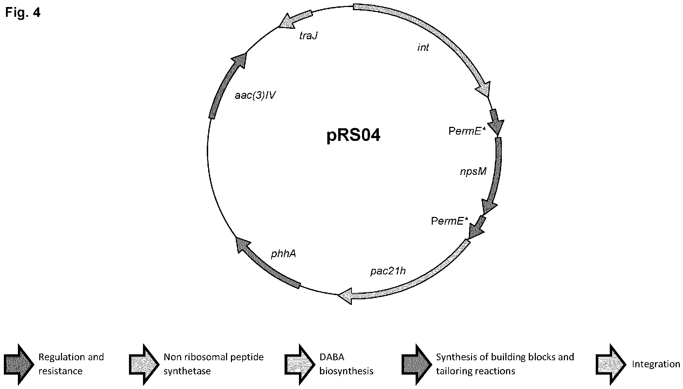

Figure 4 shows the integrative plasmid pRS04 whereby the function of the genes is shown below the circular plasmid.

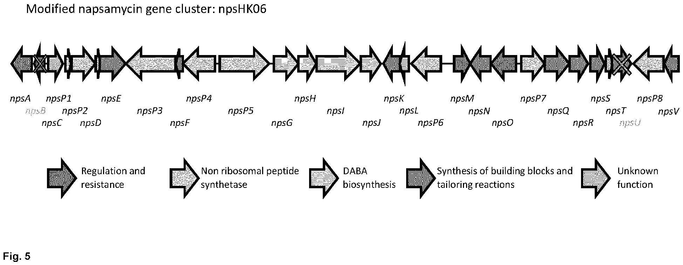

Figure 5 shows the modified napsamycin gene cluster having the designation npsHK06 whereby the function of the genes is shown below and whereby *npsB* and *npsU* are knocked out.

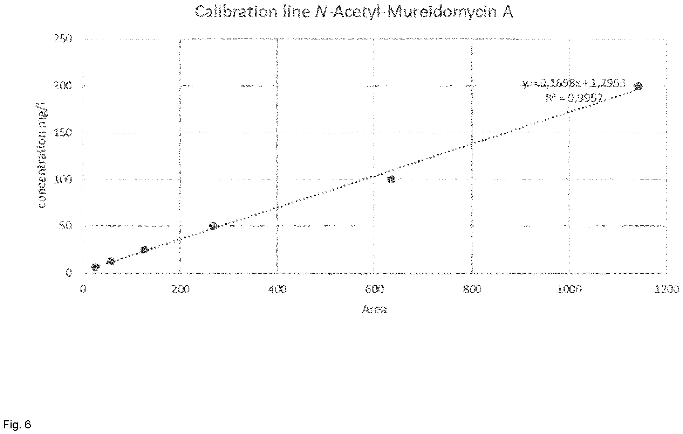

Figure 6 is a calibration curve for the determination of the concentration of mureidomycin A using N-acetyl-mureidomycin A.

Furthermore, the application contains a sequence listing showing the nucleic acid sequence of SEQ ID NO:1 - SEQ ID NO:3. The sequence listing and the information contained therein are part of the disclosure of the invention.

One strategy to allow for the production of mureidomycin A by heterologous expression of the pacidamycin gene cluster was the replacement of genes of the pacidamycin biosynthetic gene cluster pacNH01 by genes of the napsamycin biosynthetic gene cluster npsXT01. Firstly, *pac14* and *pac15* were replaced by *npsP4* and *npsP8,* respectively, in order to substitute the amino acid alanine with the amino acid methionine at position 4 (AA4) of the amino acid skeleton of UPAs. Then, *pac2* was replaced by *npsN* to allow for the installation of the amino acid glycine at position 1 (AA2) of the UPA skeleton. Subsequently, *pac21* was deleted to impede production of UPAs containing the amino acid alanine at position 2 (AA2) and complemented with plasmid pRS04, containing *pac21h, phhA* and *npsM.* Finally, *pac12* was replaced by *npsP3* to direct biosynthesis towards formation of UPAs containing meta-tyrosine at position 5 (AA5) of the of the UPA skeleton.

Subsequently, efforts were undertaken to improve production rates of uridyl peptide antibiotics (UPAs), focusing on the replacement of native promoters of the pacidamycin biosynthetic gene cluster with strong, constitutively active promoters, which would ensure full transcription of all genes situated on the respective operons. In a first analysis of the modified pacidamycin gene cluster on cosmid pacNH01, it became apparent that two sites harboring bidirectional promoters are located within the cluster: the first one between *pac3* and *pac4* and the second one between *npsP4* and *npsP8* (not shown in Fig. 2). These could be responsible for the transcription of the entire cluster except for *pac1.*

The sites of the pacidamycin gene cluster targeted by promoter replacements are displayed in Figure 2 and 3 as black arrows on the upper side of the gene clusters.

In a preferred embodiment the pacidamycin cluster has been further modified. In this embodiment promoter P15 was inserted upstream of *pac3* and P16 upstream of *pac4* (together referred to as promoter cassette 1 (PC 1). This is shown schematically in Figure 3.

Montiel et al., PNAS, Vol. 112, No. 29, pp 8953-8958 disclosed a yeast homologous recombination-based promoter engineering for the activation of silent natural product biosynthetic gene clusters. Bacteria-derived natural products and metabolites encoded by most gene clusters remain frequently uncharacterized, because they are not readily activated by commonly-used monoculture fermentation methods. Montiel et al. disclosed gene cluster activation strategies rooted in molecular cloning whereby the promoters disclosed therein, in particular promoters P15 and P16, provide a simple, cost-effective and potentially scalable tool for the characterization of the molecules encoded by such gene clusters. In this methodology, to which we herewith refer explicitly, silent gene clusters can be activated through replacement of native promoters with constitutively active synthetic promoters. Such strategy was used in the present invention.

The second strategy to improve mureidomycin A production was by heterologous expression of a modified napsamycin biosynthetic gene cluster. Deletion of *npsB* resulted in the formation of mureidomycin derivatives lacking an *N*-acetyl side chain at the N-terminus of the UPA amino acid skeleton. The additional deletion of *npsU* further restricted biosynthesis to UPAs harboring an uracil instead of a dihydrouracil and thereby directing the biosynthesis towards the formation of mureidomycin A.

The originally used *Streptomyces* strain containing the gene cluster encoded on npsXT01 mainly produces mureidomycin B and napsamycin C, while trace amounts of other derivatives like mureidomycin A and napsamycin A can be detected. The major compounds are reduced at the uracil residue. This reduction is affected by an oxireductase encoded by the gene *npsU* of the napsamycin gene cluster. Only if the gene *npsU* is knocked out by suitable genetic modifications, an increased production of mureidomycin A can be observed. Subsequently, the gene encoding for the *N*-acetyltransferase NpsB was knocked out, which further directed the biosynthesis towards mureidomycin A by eliminating the production of N-acetyl-derivatives of UPAs. It is therefore an important aspect of the present invention that key genes which allow for the production of derivatives other than mureidomycin A are precisely knocked out. This prompts not only the elimination of less bioactive derivatives, but also an enhanced production of the desired mureidomycin A.

It should be noted that the combined knockout of the genes *npsU* and *npsB* results in an increase in mureidomycin A production of up to 900 mg/l, which allows the production in a commercially acceptable way.

The examples and Figures describe preferred embodiments of the present invention.

### Examples

### Example 1

Construction of the preferred production strain *S.albus* Del14/pacHK11/pRS04

Promoter knock-ins, gene replacements and gene knockouts were conducted through λRed-mediated homologous recombination, which has been described by Gust et al. (PNAS, 2003, 100, pp 1541-1546). This strategy allows for the replacement of specific DNA sequences on plasmids and cosmids through the recombination with linear DNA fragments in the targeted strain, which is facilitated by the λRed proteins encoded on pIJ790. The linear DNA fragments in question contain selection markers and are generated by PCR, where 40 nucleotide long overhangs are added to the disruption cassette through each long PCR primer. These constitute homology arms to the sequences flanking the DNA region intended to be replaced.

For the modification of the pacidamycin gene cluster, cosmid pacNH01 was transformed into *E. coli* BW25113/pIJ790 and a single colony was subsequently inoculated into 5 mL LB medium containing chloramphenicol (Cml) and kanamycin (Kan) and incubated at 30 °C, 200 rpm overnight. The next day, 200 µL overnight culture was inoculated into 10 mL SOB medium containing 10 µL Cml, 10 µL Kan and 100 µL L-arabinose (1 M) and incubated at 30 °C, 200 rpm until the cultures reached an OD₆₀₀ of 0.4 - 0.6. Maintaining the temperature at 30 °C was crucial due to the temperature-sensitive origin of replication of plasmid pIJ790, while L-arabinose was added to induce the λRed genes. Subsequently the cells were harvested by centrifugation at 4000 rpm for 5 minutes at 4 °C. While the supernatant was discarded, the cell pellets were resuspended in 10 mL ice - cold glycerol 10 % and harvested once again by centrifugation under the same conditions. The washing process was repeated with 5 mL ice - cold glycerol 10 %, the supernatant discarded, and the cell pellet resuspended in the residual glycerol (ca. 100 µL). A 50 µL cell aliquot was electroporated with 100 ng of a PCR product consisting of the apramycin resistance gene *aac3(IV)* flanked by an I-Scel restriction site and homology sequences from the down- and upstream sequences of *pac15* of 40 bp for λRed-mediated homologous recombination. Immediately after electroporation, 500 µL SOC medium was added to the cells and mixed thoroughly, before transferring the suspension into a fresh Eppendorf tube and incubating at 37 °C and 300 rpm for 1 hour. For selection, the cell suspension was plated on LB agar containing the appropriate antibiotics (typically Kan/Apra) and incubated at 37 °C overnight. The following day, single colonies were investigated regarding the recombination by minipreparation and restriction digest or colony-PCR. Cosmid-DNA of positive candidate clones was digested with I-Scel resulting in linearized cosmid-DNA. Linearized cosmid-DNA was co-electroporated into competent and with L-arabinose induced *E. coli* BW25113/pIJ790 cells together with a linear DNA fragment consisting of *npsP8* flanked by ~ 500 bp up- and downstream sequences of *pac15.* Survivable transformants can only be obtained, if recircularization of the linearized cosmid-DNA is achieved by homologous λRed-mediated recombination with the *npsP8* DNA-fragment. Positive clones were verified by sequencing and their DNA subsequently introduced into suitable heterologous hosts by triparental conjugation.

For this purpose, the recombinant cosmid was first transformed into the methylation deficient strain *E. coli* ET12567 by electroporation and selected with chloramphenicol and kanamycin. Another strain, *E. coli* ET12567 containing the self-transmissible helper plasmid pUB307 played a crucial role, since it contains the necessary *tra* genes for the activation of the oriT of the recombinant cosmids to be transferred. 20 µL glycerol stock of *E. coli* ET12567/pUB307 and a single colony of *E. coli* ET1256 containing the recombinant cosmid were inoculated each into 5 mL of LB medium containing chloramphenicol and kanamycin and incubated at 37 °C, 200 rpm overnight. The main cultures were prepared by inoculating 200 µL overnight culture into 10 mL LB medium containing the appropriate antibiotics and incubating at 37 °C and 200 rpm until the cultures reached an OD₆₀₀ of 0.4 to 0.6. Simultaneously, *Streptomyces albus* Del14 spores were prepared by inoculating 500 µL 2xYT medium with 20 µL spore stock, conducting a heat shock in order to activate germination at 50 °C for 10 min at 300 rpm and then incubating for 2 - 4 hours at 28 °C at 300 rpm. Once the *E. coli* cells had reached the required OD₆₀₀, they were harvested by centrifugation at 4000 rpm for 5 min at 4 °C. While the supernatant was discarded, the cell pellets were resuspended in 10 mL LB medium and harvested once again by centrifugation under the same conditions. The washing process was repeated with 10 mL LB medium and afterwards the supernatant discarded once again. The cell pellet was resuspended in 1 mL LB medium, and, subsequently, mixed with the heat-shocked spores. To each 500 µL *Streptomyces* aliquot, 250 µL *E. coli* ET12567/pUB307 and 250 µL *E. coli* ET12567/cosmid were added. The vessel containing the cells was inverted carefully a few times and centrifuged briefly (approximately 10 sec) at 7000 rpm. Afterwards, 800 µL of the supernatant was removed and the residue resuspended. The obtained cell suspension was carefully plated in a dilution series of 1, 10⁻¹ and 10⁻³ on MS agar, containing 1 mL MgCl₂ (1 M). After 16 - 20 h of incubation at 30 °C, the plates were flooded completely with 1 mL of an antibiotic solution containing 25 µL kanamycin (50 mg/mL) and 25 µL nalidixic acid (25 mg/mL) per plate. Then, incubation was continued for 4 - 8 days until exconjugants appeared. Four exconjugants were transferred as a serial dilution onto separate MS - MgCl₂ plates, containing the antibiotics nalidixic acid and kanamycin and further incubated at 30 °C for 4 - 8 days. This process was repeated with the four separate exconjugants, picked from the first-generation plates and spread onto the second-generation of dilution plates. Lastly, all four exconjugants were plated confluent onto separate MS - MgCl₂ plates containing nalidixic acid and kanamycin, harvested after 4 - 8 days, verified by colony PCR and further investigated regarding mureidomycin production by LC-MS and HR-LC-MS. When the integrative plasmid pRS04 had previously been integrated into the genome, selection was additionally conducted with apramycin.

The same procedure was used for further gene replacements of *pac14* by *npsP4, pac2* by *npsN* and *pac12* by *npsP3.* For the generation of the *pac21* knockout, λRed- mediated homologous recombination with a cassette harbouring FLP-Recombinase recognition sites was used, as described by Gust et al. (2003). This resulted in cosmid pacRS03. Promoter replacement was finally achieved by the same strategy using a linear DNA-fragment containing the promoters P15 and P16 (promoter cassette PC 1) and linearized pacRS03 DNA for co-transformation into λ-Red proficient *E. coli* BW25113/pIJ790 cells to replace existing promoters between *pac3* and *pac4* by promoters P15 and P16, in accordance with the abovementioned method described for gene replacements. This final construct was termed pacHK11 and was subsequently used for triparental conjugation with *S. albus* Del14 containing plasmid pRS04, which will be described below, to obtain the strain *S*. *albus* Del14/pacHK11/pRS04.

### Example 2

### Construction of plasmid pRS04

First, genes *pac21h* and *phhA* were amplified from plasmid pSG123 provided by Rebecca Goss (University of St Andrews, St Andrews, United Kingdom) by PCR and cloned into Spel/Hindlll restriction sites of the integrative plasmid pMVM3100, obtained from Dr. Marta Vaz Mendes (IMBC University, Porto, Portugal) resulting in pRS02. Both genes, *pac21h* and *phhA,* are essential for the incorporation of *meta*-tyrosine at the amino acid position 2 of the UPA peptide skeleton and are situated under the control of the strong constitutively expressed promoter *PermE*.* In a second step, the regulatory gene of the napsamycin biosynthetic gene cluster, *npsM,* was amplified from plasmid pNH05 together with promoter *PermE** thereby introducing Avrll restriction sites at the 5' end of the PCR product. Restriction of the PCR-product with Avril then allowed for cloning into the compatible Spel sites of pRS02 to generate pRS04 (Fig. 4).

### Example 3

### Construction of S.albus Del14/npsHK06

For the modifications of the napsamycin biosynthetic gene cluster, the genes encoding for the oxidoreductase NpsU (resulting in npsHK02) and the *N*-Acetyltransferase NpsB (resulting in npsHK05) were knocked out in accordance with the previously described methodology for the knockout of *pac21.* Subsequently the region flanking the site of the *npsB* knockout was amplified and utilised in a λRed-mediated homologous recombination with the linearized cosmid npsHK02, similar to the previously described promoter replacement with the I-Scel cassette. This final construct was termed npsHK06 and was subsequently used for triparental conjugation with *S*. *albus* Del14 to obtain the strain *S. albus* Del14/pacHK06.

### Example 4

### Quantification of the produced mureidomycin A

In order to quantify mureidomycin A production, the compound *N*-Acetyl-mureidomycin A was isolated, since it closely-resembles the structure of mureidomycin A and can be used as a standard for quantification. The substance was dissolved in double distilled H₂O whereby a concentration of 200 mg/L was achieved.

The strains *S. albus* Del14/pacHK11/pRS04 and *S. albus* Del14/npsHK06 were cultivated for two days in TSB medium. This preculture was subsequently inoculated into P-medium and cultivated for seven days for the production of mureidomycin A.

To normalize measurements by cell dry weight, 1 ml of the samples was pipetted in Eppendorf tubes, centrifuged and washed with buffer. The obtained pellet was dried overnight and the dry weight of the cells was determined.

1 ml of the samples was mixed two times with n-butanol, the solvent was removed and for the measurement suspended in a water-methanol mixture. The intensity of the peaks was determined with LC-MS and semiquantitatively evaluated. The final quantitative evaluation was performed with HPLC against a concentration curve obtained with the standard *N*-Acetyl-mureidomycin A (Fig. 6).

The quantitative measurements were conducted on the HPLC utilizing *N*-Acetyl-mureidomycin A as a standard. The results obtained are shown in Table 3.

**Table 3: Production of mureidomycin A in different strains**

| | *S. albus* Del14/ npsHK06 | *S*. *albus* Del14/ npsHK06 | *S*. *albus* Del14/ pacHK11/ pRS04 | *S. albus* Del14/ pacHK11/ pRS04 |
|---|---|---|---|---|
| | [mg/L] | [mg/mg cell pellet] | [mg/L] | [mg/mg cell pellet] |
| Mureidomycin A production | 1714 mg | 902 mg | 188 mg | 38 mg |

The measurements of mureidomycin A were performed with *N*-Acetyl-mureidomycin A. The concentration of the standard *N*-Acetyl-mureidomycin is show in Figure 6.

Table 3 shows impressively that by the teaching of the present invention the yield of mureidomycin A can be enhanced by a factor of about 40 and more. This allows the production of the antibiotic mureidomycin A in a commercially acceptable way. One of the preferable properties of mureidomycin A is that it paves the way for the treatment of patients which suffer from an infection of bacteria, in particular *Pseudomonas aeruginosa,* which are resistant against many known antibiotics. In many cases patients who are infected by multi resistant bacteria die, because the bacteria are resistant against the known and commonly used antibiotics. Mureidomycin A is considered a new antibiotic which gives hope for such patients.

## Claims

1. Genetic construct comprising a genetically modified pacidamycin gene cluster pacNH01 and/or a genetically modified napsamycin npsXT01 gene cluster.

2. Genetic construct according to claim 1 **characterized in that** the pacidamycin gene cluster has been genetically engineered to enhance production of mureidomycin A wherein as a heterologous promotor P15 is inserted upstream of *pac3* and P16 upstream of *pac4* and/or that in the napsamycin biosynthetic gene cluster whereby *npsU* and *npsB* were deleted for improved production of mureidomycin A.

3. Genetic construct according to claim 1 or 2 **characterized in that** it comprises the modified pacidamycin gene cluster with an integrative plasmid pRS04 and the modified napsamycin gene cluster for improved mureidomycin A production.

4. Genetic construct according to any of claims 1 to 3 **characterized in that** in the napsamycin biosynthetic gene cluster the genes *npsU* and *npsB* are knocked out and that in the pacidamycin gene cluster *pac21* is knocked out, *pac15, pac14, pac2* and *pac12* were replaced with *npsP8, npsP4, npsN, npsP3* and heterologous promotor P15 and P16 are inserted upstream of *pac3* and *pac4.*

5. Genetic construct according to any of claims 1 to 4 **characterized in that** the construct is part of a genetic vector.

6. Genetic construct according to claim 5 **characterized in that** the vector can be a plasmid, a cosmid or a fosmid.

7. Actinomycetes strain for the improved production of mureidomycin A **characterized in that** it contains a genetic construct according to any of claims 1 to 6.

8. Actinomycetes strain according to claim 7 **characterized in that** it is a *Streptomyces albus* strain.

9. Actinomycetes strain according to any of claims 7 to 8 **characterized in that** it contains a genetic construct according to claim 4 whereby in the pacidamycin gene cluster *pac21* is knocked out, *pac15, pac14, pac2* and *pac12* were replaced with *npsP8, npsP4, npsN, npsP3* and heterologous promotor P15 and P16 are inserted upstream of *pac3* and pac4.and wherein in the napsamycin biosynthetic cluster the genes *npsU* and *npsB* are deleted.
